# EUROPÄISCHE PATENTSCHRIFT

(11) **EP 3 161 122 B1**
(45) Veröffentlichungstag und Bekanntmachung des Hinweises auf die Patenterteilung: **10.04.2019**
(21) Anmeldenummer: 15736799.6
(22) Anmeldetag: 22.06.2015
(51) Int. Cl.: C12M 3/00

(54) **EINBAURAHMEN FÜR EINEN BRUTSCHRANK**
INSTALLATION FRAME FOR AN INCUBATOR
CADRE DE MONTAGE POUR UNE ÉTUVE BACTÉRIOLOGIQUE

(30) Priorität: 26.06.2014 DE 102014212356
(43) Veröffentlichungstag der Anmeldung: 03.05.2017
(73) Patentinhaber: Memmert GmbH + Co. KG, 91126 Schwabach (DE)
(72) Erfinder: SCHMIDT, Andreas, 91623 Sachsen b. Ansbach (DE); BAYER, Heinz, 91183 Abenberg (DE)
(74) Vertreter: Tergau & Walkenhorst Patentanwälte PartGmbB
(86) Internationale Anmeldenummer: PCT/EP2015/063908
(87) Internationale Veröffentlichungsnummer: WO 2015/197511

(56) Entgegenhaltungen:
- DE-U1-202013 010 260
- FR-A1- 2 849 862
- JP-A- 2006 149 232

## Beschreibung

Die vorliegende Erfindung betrifft einen Einbaurahmen zum Einsetzen in den Innenraum eines Brutschranks. Solche Brutschränke, auch Inkubatoren genannt, werden eingesetzt, um kontrollierte Außenbedingungen für Entwicklungs- und Wachstumsprozesse zu schaffen. Im Brutschrank wird hierfür ein Mikroklima mit vorgegebenen Feuchtigkeits- und Temperaturbedingungen geschaffen. Die Erfindung betrifft insbesondere CO₂-Inkubatoren. Derartige CO₂-Inkubatoren werden beispielsweise im Bereich der Stammzellenforschung, bei der Arbeit mit Gewebekulturen und für die in vitro Fertilisation eingesetzt. Bei der In Vitro Fertilisation werden aus dem Mutterleib Eizellen entnommen und in einem Reagenzglas mit Sperma des Vaters zusammen gebracht. Im Reagenzglas findet eine Befruchtung statt. Die so geschaffenen Zygoten, also die aus der Verschmelzung der männlichen und der weiblichen Keimzelle entstehenden Diploidenzellen, aus welchen ein Lebewesen entsteht, müssen in einem Brutschrank mehrere Tage bebrütet werden. Hierfür werden die Zygoten üblicherweise in Petrischalen oder ähnlichen Behältnissen aufbewahrt und in einem Brutschrank mit Wärme, CO₂ und teilweise mit Feuchte beaufschlagt.

Hierbei ergeben sich diverse Probleme. Zunächst ist es erforderlich, die einzelnen Zygoten im Brutschrank eindeutig identifizieren zu können. Es müssen also geeignete Vorkehrungen getroffen werden, um eine Verwechselung der Zygoten miteinander mit Sicherheit ausschließen zu können. Nach dem Stand der Technik werden die Petrischalen hierfür in großen Abständen zueinander aufbewahrt.

Des Weiteren muss jede Petrischale mit den Zygoten einer bestimmten Mutter mindestens einmal am Tag aus dem Brutschrank entnommen werden, um Untersuchungen durchzuführen. Hierbei ist es problematisch, dass bei jedem Öffnungsvorgang des Brutschranks die im Brutschrank vorherrschende Atmosphäre gleichsam zusammenbricht. Dieses Zusammenbrechen der Atmosphäre im Brutschank übt Stress auf die Zygoten aus. FR 2 849 861 A1 zeigt eine Vorrichtung für Zellkulturen. Die Vorrichtung besteht aus drei Elementen, nämlich einer in sich abgeschlossenen Kammer, eine Flüssigkeitskammer mit einem Gasmischer und einer dritten Kammer, welche die Elektrik, Steuerung und Bedienungselemente der Vorrichtung aufnimmt.

Ein weiteres Problem besteht schließlich in der Notwendigkeit, den gesamten Brutschrank sterilisieren zu müssen, sobald mehrere zueinander gehörende Zygoten nach dem Bebrüten dauerhaft aus dem Brutschrank entfernt werden. Während dieses Sterilisierungsvorgangs müssen deshalb die im Brutschrank verbleibenden Petrischalen mit den übrigen Zygoten in einen anderen Brutschrank aufwendig überführt werden.

Ausgehend hiervon liegt der Erfindung die Aufgabe zugrunde, einen Brutschrank hinsichtlich seiner Gebrauchseigenschaften zu verbessern. Diese Aufgabe ist durch die Merkmalskombination des Anspruchs 1 in erfinderischer Weise gelöst. Die rückbezogenen Ansprüche beinhalten teilweise vorteilhafte und teilweise für sich selbst erfinderische Weiterbildungen dieser Erfindung.

Die Erfindung baut auf der Grundüberlegung auf, den Innenraum eines vorhandenen Brutschranks durch mehrere offene Lagerfächer zu unterteilen. Diese Unterteilung soll mithilfe eines Einbaurahmens erfolgen, welcher sämtliche Unterteilungen trägt. Der Einbaurahmen kann nämlich so als Ganzes aus dem Brutschrank mit einem Griff entfernt werden. Beim Sterilisieren kann die Beschickung eines Brutschranks, also die im Brutschrank befindlichen Reagenzien durch das Überführen des Einbaurahmens in einen anderen Brutschrank mit einem Griff in den anderen Brutschrank überführt werden.

Bei einem Brutschrank schließt die Haupttür den Innenraum des Brutschranks gegenüber der Außenwelt luftdicht ab. Wird die Haupttür jedoch geöffnet, kollabiert das im Brutschrank vorherrschende Mikroklima wie eingangs erwähnt. Nach der Erfindung ist deshalb eine die Frontöffnung des Innenraums hinter der Haupttür ausfüllende Frontplatte vorgesehen. Die Frontplatte hält so das Mikroklima im Schrank zumindest für einen gewissen Zeitraum aufrecht auch wenn die Haupttür geöffnet ist. Der Einbaurahmen isoliert so den Inhalt des Brutschranks mit der Frontplatte nach außen, während der Inhalt auf den Tablaren separiert im Innenraum offen gelagert ist. Es bleibt also der einheitliche Innenraum mit seinem Mikroklima für den im Brutschrank auf den Tablaren gelagerten Inhalt erhalten. Dies ermöglich es auch den Brutschrank mit einliegendem Einbaurahmen gemeinsam als Ganzes in einem Arbeitsgang zu sterilisieren. Während des Sterilisierungsvorgangs müssen die im Brutschrank behandelten Reagenzien vollständig entfernt werden.

Um einzelne Zygoten oder einzelne Reagenzien des Beschickungsguts entnehmen zu können, ohne dass das Mikroklima im Brutschrank kollabiert, sind in der Frontplatte Durchtrittsöffnungen vorgesehen, welche separat geöffnet und geschlossen werden können. Hinter den Duchtrittsöffnungen in Richtung auf den Innenraum des Brutschranks sind Tablare angeordnet zur Aufnahme des jeweiligen Beschickungsguts. Auf diese Weise ist es möglich, stets gezielt nach einer Petrischale zu suchen und nur die dem Tablar der jeweiligen Petrischale zugeordnete Durchtrittsöffnung zu öffnen und die Petrischale zu entnehmen. Die übrigen Tablare bleiben während dieser Entnahme verschlossen. Im Brutschrank bleiben so der CO₂-Gehalt, die Temperatur, der Sauerstoffgehalt und weitere Parameter der Atmosphäre wie die relative Feuchte vom Öffnen der einzelnen Durchtrittsöffnung mehr oder weniger unbeeinflusst. Die sogenannten Erholzeiten, also die Zeit zur Wiedererreichung der eingestellten Sollwerte bzw. Sollparameter, wird somit auf ein Minimum reduziert. Dadurch dass der Innenraum in dem Bereich hinter der Frontplatte, welcher der Haupttür abgewandt ist, nach wie vor ein einziger durchgehender Raum ist, lassen sich die vorerwähnten Parameter wie der CO₂-Gehalt, die Temperatur, der Sauerstoffgehalt sowie die relative Feuchte nach wie vor zentral am Brutschrank regeln. Es kann also auch ein bereits vorhandener Brutschrank eines Herstellers mit dem erfindungsmäßigen Einbaurahmen versehen werden bei vollständiger Erhaltung seiner Funktionalität.

In vorteilhafter Ausgestaltung isoliert die Frontplatte den Innenraum des Inkubators thermisch gegenüber der Außenwelt. In weiterer vorteilhafter Ausgestaltung ist die Frontplatte hierfür sandwichartig aufgebaut mit einem Kunststoffkern aus thermisch isolierendem Material. Dieser Kunststoffkern ist in vorteilhafter Ausgestaltung mehrschichtig aufgebaut. Alternativ kann auch eine doppelwandige Frontplatte mit einem thermisch isolierenden Vakuum vorgesehen sein. Die Frontplatte funktioniert dann ähnlich wie eine Isolierkanne und isoliert den Innenraum des Brutschranks gegenüber der Außenwelt.

Die isolierende Frontplatte allgemein hat den Vorteil, dass durch das Öffnen der Haupttür keine Veränderung des Mikroklimas im Brutschrank erfolgt. Erst durch das Öffnen einer Durchtrittsöffnung zum Innenraum ändert sich das Mikroklima im Innenraum geringfügig. Gegenüber dem Stand der Technik ist diese Änderung jedoch vernachlässigbar, was deutlich weniger Stress für das im Innenraum des Brutschranks befindliche Beschickungsgut bedeutet.

In weiterer Ausgestaltung sind die Durchtrittsöffnungen durch in ihrem Innenaufbau dem Innenaufbau der Frontplatte entsprechende Öffnungsplatten verschlossen. Dieser identische oder ähnliche Innenaufbau von Frontplatte und Öffnungsplatten hat zum Einem den Vorteil, dass auch die Öffnungsplatten den Innenraum des Brutschranks gegenüber der Außenwelt isolieren. Zum Anderen führt dies zu einer glatten durchgehenden Frontwand auf der Frontplatte.

Um die Entnahme des Beschickungsguts zu vereinfachen sind die Tablare verschieblich an der Frontplatte gelagert. Nach dem Öffnen der Durchtrittsöffnungen, insbesondere dem Öffnen der Öffnungsplatten können die Tablare einfach in Richtung auf den Außenraum des Brutschranks herausgezogen werden, das Beschickungsgut kann entnommen werden und das Tablar entweder mit oder ohne Beschickungsgut wieder in Richtung auf den Innenraum in seine Ausgangsstellung verschoben werden, um anschließend die Öffnungsplatte und damit die Frontplatte wieder zu verriegeln. Die Tablare können hierfür motorisch angetrieben sein.

Besonders einfach ist es natürlich, die Öffnungsplatten an den der Frontplatte zugewandten Enden der Tablare zu fixieren, so dass die Tablare und die Frontplatten nach Art von Schubladen an der Frontplatte gelagert sind. Die Schubladen können dann einfach aufgezogen und wieder eingeschoben werden. In weiterer bevorzugter Ausgestaltung sind an den Schubladen ausschwenkbare Griffleisten vorgesehen, welche im ausgeschwenkten Zustand untergriffen werden können. Im eingeschwenkten Zustand dieser Griffleisten ist die Frontplatte wiederrum völlig eben. Die Frontplatte kann so sehr nah an der Rückwand der Haupttür fixiert sein und benötigt folglich einen nur geringen Einbauraum im Brutschrank.

In weiterer vorteilhafter Ausgestaltung bestehen die Tablare aus einem Halterahmen und einem oder mehreren in den Halterahmen eingesetzten Auflageplatten. Auf diese Weise können die Halterahmen kostengünstig als Blechbiegeteile hergestellt werden. In diese Halterahmen können dann auch besonders ausgestaltete Auflageplatten eingesetzt werden. Die Auflageplatten können beispielsweise aus einem besonders gut wärmeleitenden Werkstoff bestehen. Auch ist es möglich, in die Aufnahmeplatten Konturen einzubringen, welche die Aufnahme bestimmter Trägermedien für das Beschickungsgut besonders begünstigen. So ist es beispielsweise möglich, ringförmige Nuten in die Aufnahmeplatten einzubringen. In diese ringförmigen Aufnahmenuten können aus dem Schalenboden einer Petrischale vorspringende umlaufende Ränder eingreifen, so dass die Petrischale mit ihrem Schalenboden direkt auf der Auflageplatte aufliegt, was die Wärmeübertragung begünstigt. Üblicherweise dienen die umlaufenden kreisförmigen Ränder zur Verhinderung eines Kontakts des Petrischalenbodens mit anderen Substanzen, um ein Verkratzen der Petrischale zu verhindern. Dieses Verkratzen kann durch eine gezielte Werkstoffauswahl bei den Auflageplatten jedoch wirksam verhindert werden.

Besonders vorteilhaft ist es schließlich, an der dem Innenraum zugewandten Rückseite der Frontplatte seitlich zwei U-förmige Stützbügel vorzusehen. Diese Stützbügel sind mit den Stirnseiten ihrer U-Schenkel an der Frontplatte fixiert. Der dem Schrankboden des Brutschranks zugewandten U-Schenkel kann beim Einschieben des Einbaurahmens als Gleitfuß wirken. Besonders vorteilhaft ist es zusätzliche Gleitkufen auf der Außenseite des unteren U-Schenkels vorzusehen. Diese Gleitkufen können als Peek-Füße oder Teflonfüße ausgestaltet sein. Diese Peek-Füße oder Teflonfüße bilden eine Art Gleitlager gemeinsam mit dem Brutschrankboden aus. Auf diese Weise ist es dann sehr einfach möglich, den Einbaurahmen in einem Brutschrank einzuschieben bzw. aus einem Brutschrank herauszuziehen. Besonders erleichtert dies das Sterilisieren eines Brutschranks. Zum Sterilisieren muss dann nur der gesamte Einsatz aus dem Brutschrank herausgezogen und in einen anderen Brutschrank überführt werden, um den Brutschrank anschließend sterilisieren zu können. Auch diese einfache Handhabbarkeit des Brutschrankinhalts mit einem Handgriff vermindert die negativen Auswirkungen auf das Beschickungsgut. Wie oben erwähnt, kann der leere Brutschrank ohne Beschickungsgut auch gemeinsam mit dem Einsatz in einem Arbeitsgang sterilisiert werden.

Besonders vorteilhaft ist es, einen Inkubator von vornherein mit einem erfindungsmäßigen Einbaurahmen zu kombinieren. Es ist aber auch möglich, einen Einbaurahmen gezielt in Anpassung an einen vorhandenen Brutschrank auszugestalten, um den Brutschrank künftig gemeinsam mit dem Einbaurahmen als Kombinationsteil ausliefern zu können oder um vorhandene Brutschränke mit dem Einbaurahmen nachzurüsten.

Bei dem erfindungsmäßigen Brutschrank ist also die Frontplatte nach Art eines tragenden Skeletts wirksam. An dieser tragenden Frontplatte, die zugleich den Innenraum gegenüber der Außenwelt wirksam isoliert, sind die einzelnen Tablare angeordnet. In der Praxis hat sich eine Anordnung von sechs oder acht Tablaren bewährt, welche in zwei Spalten nebeneinander und in drei oder vier Zeilen übereinander angeordnet sind.

Zur Verhinderung der Verwechslungsgefahr der Reagenzien sind sowohl die Reagenzien als auch die Tablare oder die Auflageplatten entsprechend einzeln beschriftet. Alternativ ist es auch möglich, die Reagenzien und die Auflageplatten farbig zu kodieren. In weiterer Ausgestaltung ist es denkbar, neben den Öffnungsplatten Displays anzuordnen, welche die Patientendaten bzw. Reagenzdaten der auf den Tablaren gelagerten Reagenzen anzeigen, welche diesen Öffnungsplatten zugeordnet sind. Auch kann ein Scanner in die Frontplatte integriert sein, welcher digitale Codes, z.B. Barcodes oder QR-Codes einliest und das zum digitalen Code zugehörige aus Öffnungsplatte und Tablar gebildete Fach freigibt oder vorzugsweise motorisch öffnet.

Bei der Ausführungsform mit den schubladenartig kombinierten Tablaren und Öffnungsplatten ist es vorteilhaft eine zusätzliche Verriegelung derart vorzusehen, dass stets nur ein Auszug herausgezogen und damit geöffnet sein kann. Auf diese Weise ist sichergestellt, dass durch das Öffnen mehrerer Auszüge nicht das Mikroklima im Brutschrank ungewollt kollabiert.

Anhand der Zeichnungsfiguren ist die Erfindung mit weiteren Einzelheiten erläutert. Es zeigen:

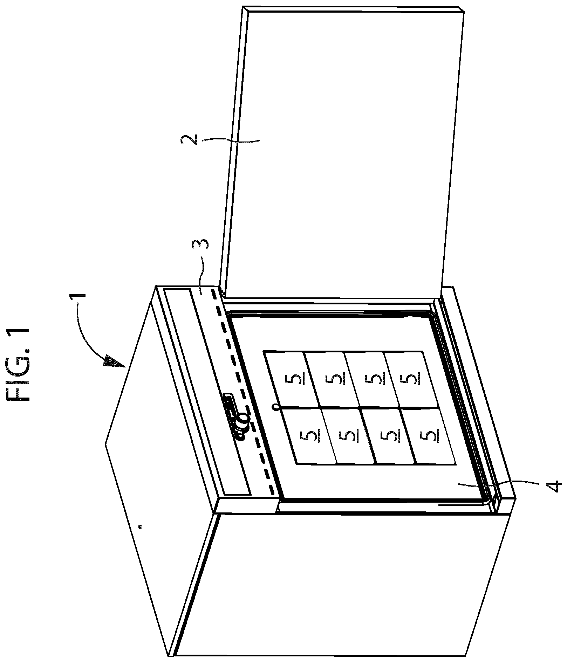

- Fig. 1: Eine perspektivische Ansicht eines erfindungsmäßigen Inkubators mit eingebautem Einbaurahmen und geöffneter Haupttür,
- Fig. 2: eine geschnittene dreidimensionale Ansicht des Inkubators aus Fig. 1,
- Fig. 3a: einen Einbaurahmen mit acht Öffnungsplatten,
- Fig. 3b: einen Einbaurahmen mit sechs Öffnungsplatten,
- Fig. 4: ein modular aufgebautes Tablar mit eingelegten Auflageplatten,
- Fig. 5: das Tablar aus Fig. 4 mit zwei entfernten Auflageplatten,
- Fig. 6: ein nach Art einer Schublade aufgebautes Tablar,
- Fig. 7: das Tablar gemäß Fig. 6 mit zwei herausgenommenen Auflageplatten,
- Fig. 8: eine weitere Ausgestaltung eines Tablars,
- Fig. 9: eine weitere Ausgestaltung eines nach Art einer Schublade aufgebauten Tablars,
- Fig. 10: eine weitere Ausführungsform eines schubladenartig aufgebauten Tablars,
- Fig.11: eine Ausführungsform eines schubladenartig aufgebauten Tablars gemäß Fig. 5 mit fest installierten Kameramodul,
- Fig. 12: eine Ausführungsform eines schubladenartig aufgebauten Tablars gemäß Fig. 8 mit unten angeflanschtem Kameramodul,
- Fig. 13: eine Ausführungsform eines schubladenartig aufgebauten Tablars gemäß Fig. 4 mit einem in eine Auflageplatte integrierten Kameramodul,
- Fig. 14: eine vorteilhafte Ausgestaltung einer Öffnungsplatte und
- Fig. 15: mehrere Ausführungsbeispiele des inneren Aufbaus der Frontplatte.

Der Brutschrank 1 weist einen seinen Innenraum 6 umschließenden Korpus auf, welcher an seiner Vorderseite von einer Haupttür 2 verschlossen ist. Oberhalb der Haupttür 2 ist das kombinierte Bedien- und Anzeigefeld 3 angeordnet. Hinter der geöffneten Haupttür 2 erkennt man die die gesamte Frontöffnung des Brutschranks 1 ausfachende Frontplatte 4 und die mit der Frontplatte 4 in einer Ebene verlaufenden Öffnungsplatten 5, welche die ihnen jeweils zugeordneten Durchtrittsöffnungen verschließen.

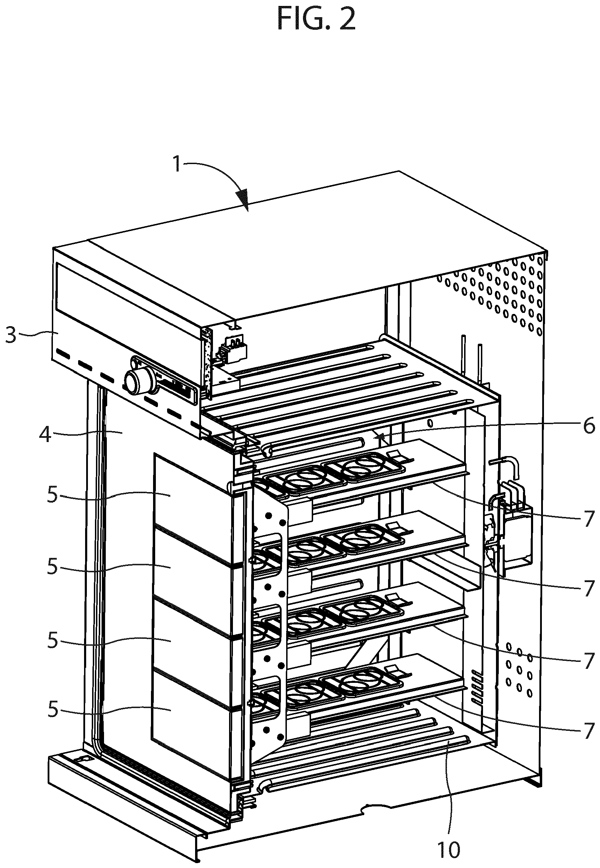

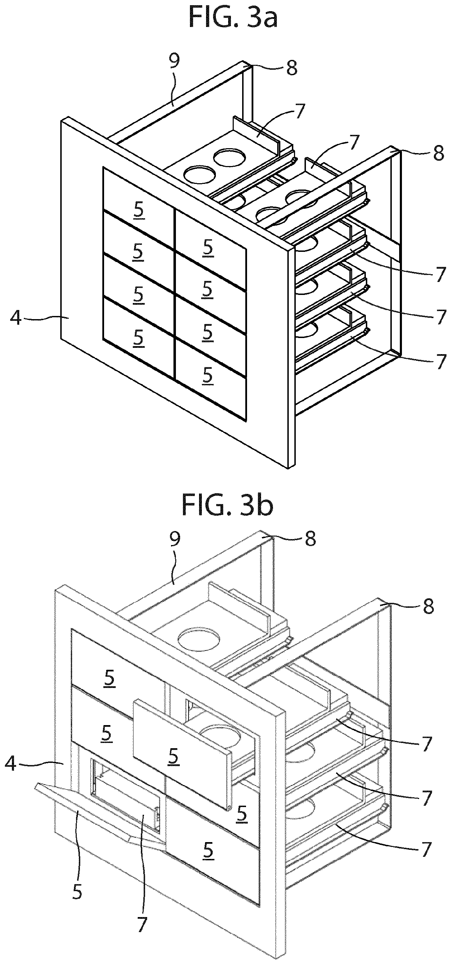

In der Darstellung der Fig. 2 ist der vom Körper des Brutschrank 1 umschlossene Innenraum 6 des Brutschranks 1 erkennbar. In den Innenraum 6 ragen einzelne Tablare 7 hinein. Die Tablare 7 bilden mit den Öffnungsplatten 5 im Ausführungsbeispiel der Fig. 2 jeweils eine schubladenartige Baueinheit. Fig. 3a zeigt wiederum eine Frontplatte 4 mit vier jeweils übereinander angeordneten Öffnungsplatten 5, welche zweireihig nebeneinander angeordnet sind. Es sind wieder Tablare 7 erkennbar. Ferner sind in Fig. 3 zwei U-förmige Stützbügel 8 erkennbar, die mit den Stirnseiten ihrer U-Schenkel 9 an der Rückseite der Frontplatte 4 befestigt sind. Der jeweils untere U-Schenkel 9 gleitet beim Einbau in den Brutschrank 1 nach Art einer Gleitkufe auf dem Brutschrankboden 10. In Fig. 3b ist oben rechts ein Tablar 7 mit einer Öffnungsplatte 5 gezeigt, welche nach Art einer Schublade zusammen wirken. Unten links ist ein Tablar 7 gezeigt, welches von einer schwenkbar an der Frontplatte 4 gelagerten Öffnungsplatte 5 verschlossen ist. Aus der Darstellung der Fig. 2 ist gut erkennbar, dass die Frontplatte 4 den Innenraum 6 nach außen hin luftdicht abschließt, während die Tablare 7 völlig frei zugänglich im Innenraum 6 gelagert sind. Auf diese Weise kann das Mikroklima im Brutschrank 1 über das Bedien- und Anzeigefeld 3 leicht gesteuert werden.

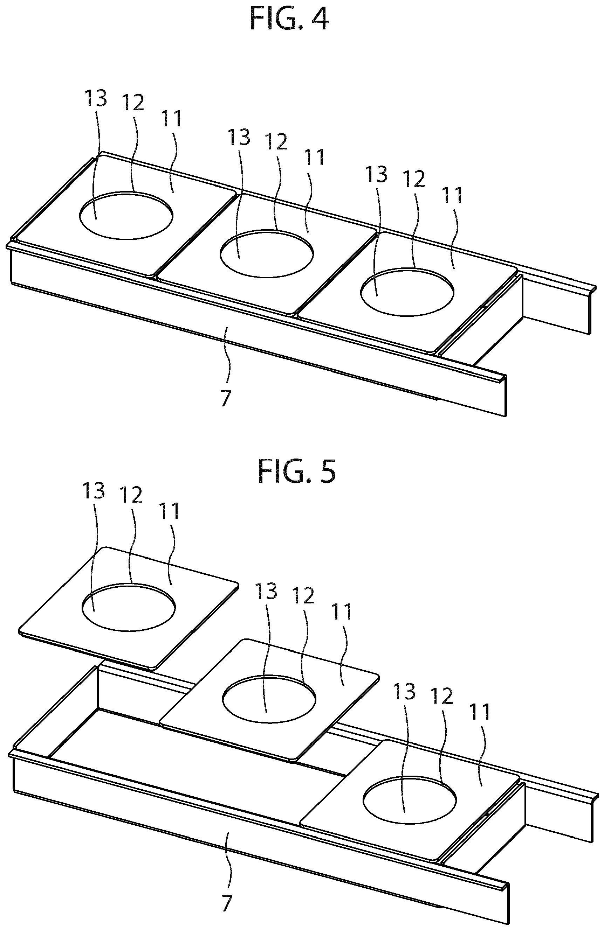

Fig. 4 und Fig. 5 zeigen ein wannenartiges Tablar 7 mit den die Aufnahmewanne 23 abdeckenden Auflageplatten 11. Auf die Auflageplatten 11 werden die Petrischalen mit den einzelnen Reagenzien aufgestellt. Die Auflageplatten 11 weisen kreisförmige Nuten 12 auf, welche Auflageböden 13 umsäumen. Auf die Auflageböden 13 sind Petrischalen mit ihren Böden aufstellbar, indem die kreisrunden Stellfüße der Petrischalen in die Nuten 12 eingreifen um so den direkten Kontakt der Auflageböden 13 mit den Böden der Petrischalen herzustellen.

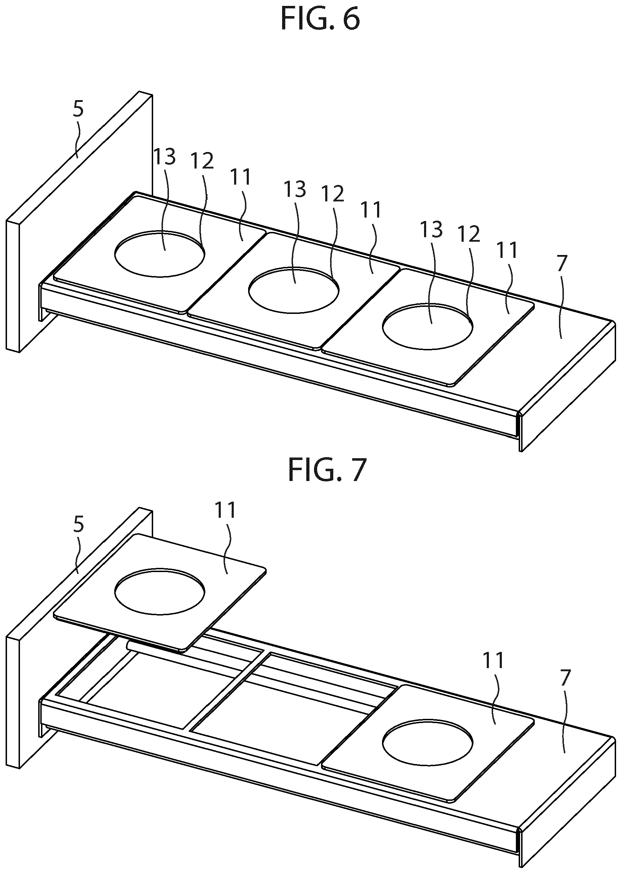

Fig. 6 zeigt eine Ausführungsform der Tablare 7 mit einem als Blechbiegeteil ausgestalteten Rahmen, in welchen wiederum Auflageplatten 11 eingesetzt sind. Die Auflageplatten 11 entsprechen in ihrem Aufbau in Fig. 4 und Fig. 5 bereits beschriebenen Auflageplatten 11.

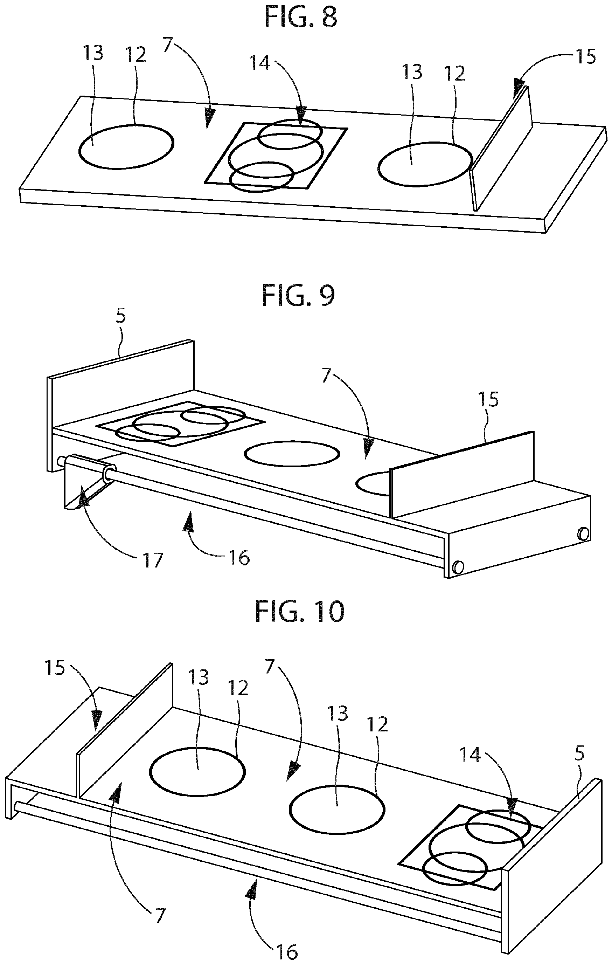

Fig. 8 zeigt ein Tablar 7 mit Nuten 12, welche wiederum Auflageböden 13 umgrenzen. Fig. 8 zeigt darüber hinaus noch mehrere Ausfräsungen 14 in der Oberseite des Tablars 7. Die Ausfräsungen 14 dienen zur Aufnahme von Reagenzbehältern unterschiedlicher Geometrie. Weiterhin erwähnenswert ist in Fig. 8 die Verschlussbarriere 15. Die Verschlussbarriere 15 verschließt bei vollständig ausgezogenem Tablar 7 zur Entnahme der Reagenzien die dem Tablar 7zugeordnete Durchtrittsöffnung in der Frontplatte 4. Die Verschlussbarriere 15 dient also dazu, auch bei ausgezogenem Tablar 7 die Durchtrittsöffnung zu verschließen, um das Mikroklima im Brutschrank 1 während der Manipulation der Reagenzien möglichst nicht zu beeinträchtigen. In anderer Ausgestaltung können auch mehrere Verschlussbarrieren 15 mit Abstand zueinander auf dem Tablar 7 angeordnet sein, um die Durchtrittsöffnung auch bei nur teilweise ausgezogenem Tablar 7 zu verschließen. Die Verschlussbarrieren 15 segmentieren hierbei die Tablare 7 in separate Zonen, um die Reagenzien voneinander zusätzlich zu trennen und einzeln entnehmen zu können.

Fig. 9 zeigt ein als Schublade mit angeformter Öffnungsplatte 5 ausgestaltetes Tablar 7, welches wiederum eine Verschlussbarriere 15 aufweist. Das Tablar 7 weist eine Stangenführung 16 mit einem zugehörigen Gleitlager 17 auf. Fig. 10 zeigt eine Kombination des Tablars 7 aus Fig. 8 und des schubladenartigen Aufbaus aus Fig. 9.

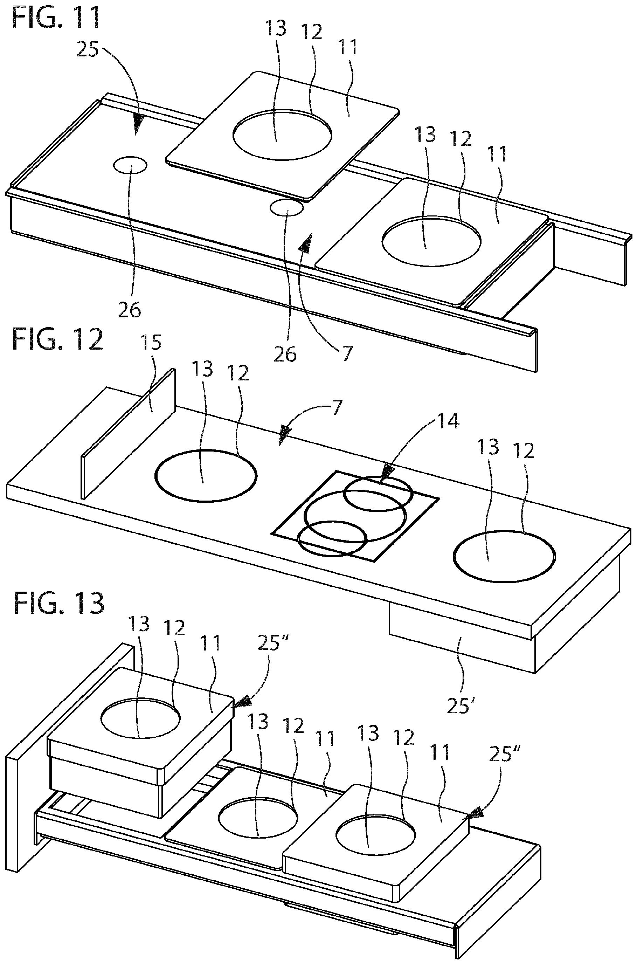

Fig. 11 zeigt das wannenartige Tablar 7 aus Fig 5. mit den die Aufnahmewanne 23 abdeckenden Auflageplatten 11. Auf die Auflageplatten 11 werden die Petrischalen mit den einzelnen Reagenzien aufgestellt. Die Auflageplatten 11 weisen kreisförmige Nuten 12 auf, welche Auflageböden 13 umsäumen. Auf die Auflageböden 13 sind Petrischalen mit ihren Böden aufstellbar, indem die kreisrunden Stellfüße der Petrischalen in die Nuten 12 eingreifen um so den direkten Kontakt der Auflageböden 13 mit den Böden der Petrischalen herzustellen. Im Tablar 7 ist ferner ein Kameramodul 25 angeordnet. Dieses Kameramodul 25 weist zwei Kameraobjektive 26 auf. Jedes Kameraobjektiv 26 ist zentral unter dem Mittelpunkt des darüber liegenden Auflagebodens 13 angeordnet und kann die Reagenz in der auf dem Auflageboden 13 aufliegenden, nicht dargestellten Petrischale aufnehmen. Das Kameramodul 25 ermöglicht die kontinuierliche Beobachtung der Reagenzien, ohne diese aus dem Brutschrank 1 entnehmen zu müssen.

Fig. 12 zeigt das Tablar 7 gem. Fig. 8 mit Nuten 12, welche Auflageböden 13 umgrenzen und mehreren Ausfräsungen 14 in der Oberseite des Tablars 7. Die Ausfräsungen 14 dienen zur Aufnahme von Reagenzbehältern unterschiedlicher Geometrie. Am Tablar 7 ist unterhalb eines Auflagebodens 13 ein Kameramodul 25' angeflanscht. Dieses Kameramodul 25' hat ein auf den Auflageboden 13 ausgerichtetes Kameraobjektiv 26 zur Beobachtung und Aufnahme der auf den Aufnahmeboden 13 aufgestellten Reagenz.

Fig. 13 zeigt schließlich ein Kameramodul 25" mit fest aufgesetzter Auflageplatte 11. Die Auflageplatte 11 und das Kameramodul 25" bilden hier ein integrales Bauteil. Die Auflageplatten 11 weisen wiederum kreisförmige Nuten 12 auf, welche Auflageböden 13 umsäumen. Auf die Auflageböden 13 sind Petrischalen mit ihren Böden aufstellbar, indem die kreisrunden Stellfüße der Petrischalen in die Nuten 12 eingreifen um so den direkten Kontakt der Auflageböden 13 mit den Böden der Petrischalen herzustellen. Zentral unter dem Mittelpunkt des Auflagebodens 13 ist wiederum das Kameraobjektiv 26 angeordnet und kann die in der auf dem Auflageboden 13 aufliegenden, nicht dargestellten Petrischale befindliche Reagenz aufnehmen. In Fig. 13 ist ferner die Möglichkeit dargestellt, Auflageplatten 11 mit integriertem Kameramodul 25" und Auflageplatten 11 ohne Kameramodul 25? miteinander zu kombinieren. In Fig. 13 weisen die beiden randseitigen Auflageplatten 11 Kameramodule 25" auf, während die mittlere Auflageplatte 11 ohne Kameramodul ist.

Die Kameramodule 25, 25',25" ermöglichen es somit, die Reagenzien bei verschlossenem Brutschrank 1 kontinuierlich zu beobachten. Dies hat ein noch selteneres Öffnen des Brutschranks 1 und deutlich weniger Stress für die Reagenzien zur Folge.

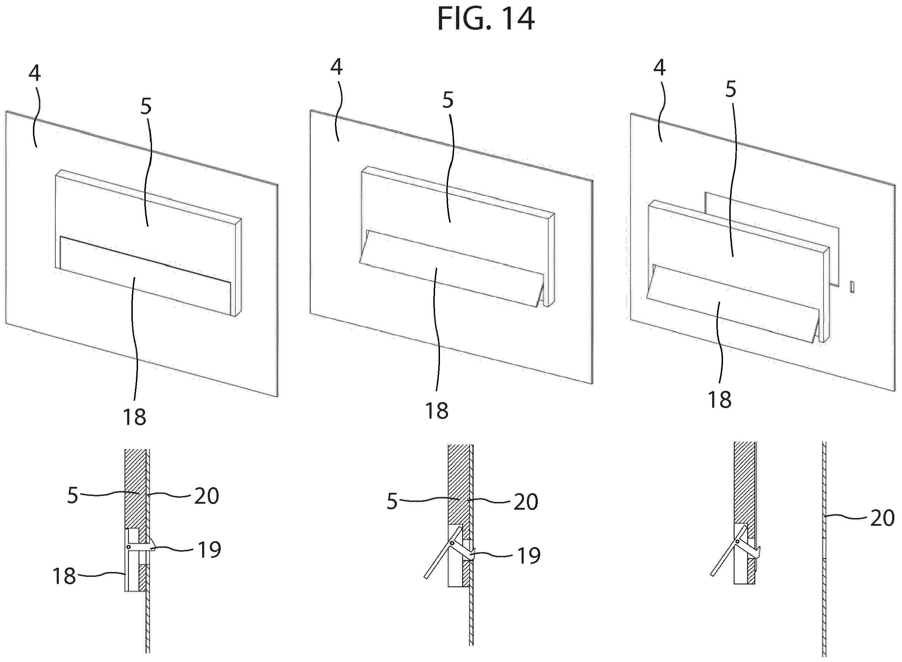

Fig. 14 zeigt eine Ausschnittsdarstellung einer Frontplatte 4 mit einer Öffnungsplatte 5. Unten an der Öffnungsplatte 5 ist eine Griffleiste 18 angeformt. Die Griffleiste 18 ist in der Figur links in geschlossenem Zustand dargestellt. Hierbei hintergreift ein Fanghaken 19 die Rückwand 20 der Frontplatte 4. Auf diese Weise ist Öffnungsplatte 5 an der Frontplatte 4 fest verriegelt. Zum Öffnen wird die Griffleiste 18 schräg gestellt, was in der mittleren Darstellung von der Fig. 11 erkennbar ist. Infolge der Schrägstellung der Griffleiste 18 lässt sich die Griffleiste 18 zum einem gut untergreifen und zum anderen entriegelt der Fanghaken 19 seine Hintergriffstellung mit der Rückwand 20 der Frontplatte 4. In der rechten Darstellung der Fig. 11 ist erkennbar, dass mit der Öffnungsplatte 5 das schubladenartige Tablar 7 aus dem Innenraum 6 des Brutschranks 1 herausgezogen werden kann.

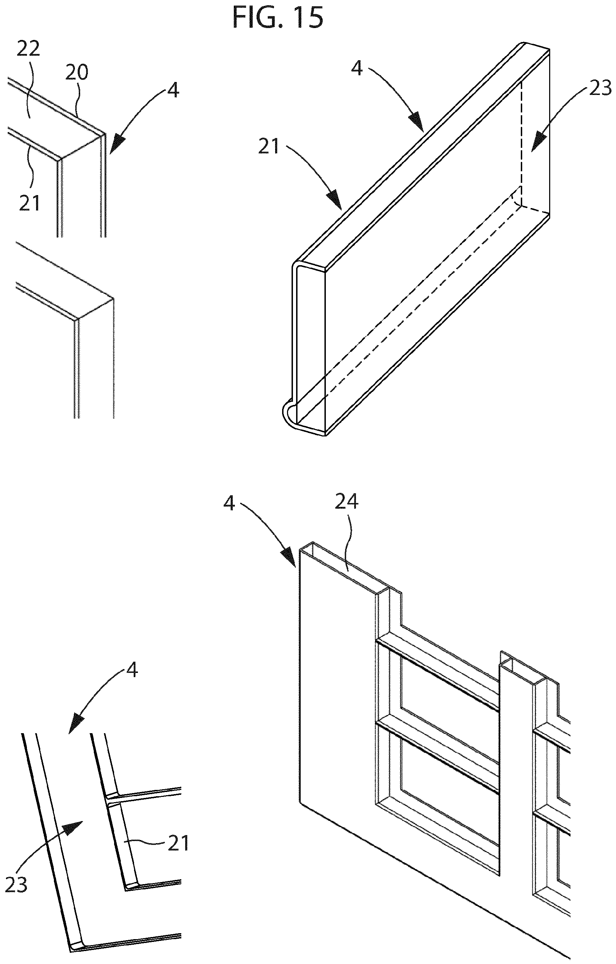

Fig. 15 zeigt schließlich einige Ausführungsbeispiele des Aufbaus der Frontplatte 4. In Fig. 12 links oben ist eine Frontplatte 4 mit einer Rückwand 20 und einer Vorderwand 21 gezeigt. Zwischen die Rückwand 20 und die Vorderwand 21 ist eine Isolierplatte 22 zwischengebaut. In Fig. 12 rechts oben ist eine wannenartige Vorderwand 21 einer Frontplatte 4 gezeigt, in deren Aufnahmewanne 23 wiederum isolierendes Material eingebracht werden kann. Unten rechts in Fig. 12 ist schließlich eine Konstruktion einer Frontplatte 4 gezeigt, mit einem Hohlraum 24 zur Einbringung eines Vakuums, um nach Art einer Isolierkanne zu isolieren.

Die Wirkungsweise des Brutschranks 1 ist folgende:
Zur Füllung oder zur Entnahme des Brutschranks 1 mit Beschickungsgut wird die Haupttür 2 geöffnet. Nach dem Öffnen der Haupttür 2 wird ein Tablar 7 mithilfe der dem Tablar 7 zugeordneten Öffnungsplatte 5 herausgezogen. Hierbei ist eine Verriegelung derart vorgesehen, dass die übrigen Tablare 7 nicht aus ihrer Position im Brutschrank 1 herausgezogen werden können. Ein derartiger Verriegelungsmechanismus ist beispielsweise von Büroschreibtischen bekannt.

Sobald die Reagenzien des Beschickungsguts entnommen oder aufgestellt sind, wird das Tablar 7 wieder in seine Ausgangsposition in den Innenraum 6 des Brutschranks 1 eingeschoben und die Haupttür 2 des Brutschranks 1 wiederum verschlossen.

Zum Sterilisieren des Brutschranks 1 wird der gesamte in Fig. 3 gezeigte Einbaurahmen aus dem Brutschrank 1 entfernt und in einen anderen Brutschrank 1 überführt. Der Ursprungs-Brutschrank 1 kann sodann sterilisiert werden.

### Bezugszeichenliste

- 1: Brutschrank
- 2: Haupttür
- 3: Bedien- und Anzeigefeld
- 4: Frontplatte
- 5: Öffnungsplatte
- 6: Innenraum
- 7: Tablar
- 8: Stützbügel
- 9: U-Schenkel
- 10: Brutschrankboden
- 11: Auflageplatte
- 12: Nut
- 13: Auflageboden
- 14: Ausfräsung
- 15: Verschlussbarriere
- 16: Stangenführung
- 17: Gleitlager
- 18: Griffleiste
- 19: Fanghaken
- 20: Rückwand
- 21: Vorderwand
- 22: Isolierplatte
- 23: Aufnahmewanne
- 24: Hohlraum
- 25, 25',25": Kameramodul
- 26: Kameraobjektiv

## Patentansprüche

1. Einbaurahmen zum Einsetzen in den Innenraum (6) eines Brutschrankes (1)
• mit einer die Frontöffnung des Innenraumes (6) ausfachenden Frontplatte 4,
• mit mindestens zwei verschließbaren Durchtrittsöffnungen zum Innenraum (6) in der Frontplatte (4) und
• mit sich an die Durchtrittsöffnungen in Richtung auf den Innenraum (6) anschließenden Tablaren (7) zur Aufnahme des zu behandelnden Beschickungsgut.

2. Einbaurahmen nach Anspruch 1
**dadurch gekennzeichnet,**
**dass** die Frontplatte (4) den Innenraum (6) luftdicht isoliert.

3. Einbaurahmen nach Anspruch 2
**dadurch gekennzeichnet,**
**dass** die Frontplatte (4) sandwichartig aufgebaut ist mit einem Kunststoffkern (= Isolierplatte (22)) aus thermisch isolierendem Material.

4. Einbaurahmen nach Anspruch 2
**gekennzeichnet durch**
eine doppelwandige Frontplatte (4) mit einem thermisch isolierenden Vakuum zwischen ihren Wandungen (20, 21).

5. Einbaurahmen nach einem der Ansprüche 1 - 4
**dadurch gekennzeichnet**
**dass** die Durchtrittsöffnungen jeweils durch eine in ihrem Innenaufbau der Frontplatte (4) entsprechende Öffnungsplatte (5) verschlossen sind.

6. Einbaurahmen nach einem der Ansprüche 1 - 5
**dadurch gekennzeichnet,**
**dass** die Tablare (7) in Richtung auf den Innenraum (6) längsverschieblich an der Frontplatte (4) gelagert sind.

7. Einbaurahmen nach Anspruch 6
**dadurch gekennzeichnet,**
**dass** die Öffnungsplatten (5) an den der Frontplatte (4) zugewandten Enden der Tablare (7) fixiert sind derart, dass jeweils eine Frontplatte (4) und ein Tablar (7) eine Schublade miteinander ausbilden.

8. Einbaurahmen nach einem der Ansprüche 1 - 7
**dadurch gekennzeichnet,**
**dass** die Tablare (7) jeweils aus einem Halterahmen und aus einer oder mehreren in den Halterahmen einsetzbaren Auflageplatten (11) bestehen.

9. Einbaurahmen nach einem der Ansprüche 1 - 8
**gekennzeichnet durch**
zwei U-förmige, mit den Stirnseiten ihrer U-Schenkel (9) an der dem Innenraum (6) zugewandten Seite der Frontplatte (4) fixierten Stützbügeln (8) derart, dass der dem Schrankboden (10) des Brutschranks (1) zugewandte U-Schenkel (9) auf seiner dem Brutschrankboden (10) zugewandten Außenseite Gleitkufen trägt.

10. Brutschrank (1) mit einem in seinem Innenraum (6) eingebauten Einbaurahmen nach einem der Anspruch 1 - 9.

## Claims

1. Installation frame for insertion into the interior space (6) of an incubator (1),
• having a front plate (4) which forms the front opening of the interior space (6),
• having at least two closable passage openings to the interior space (6) in the front plate (4), and
• having shelves (7) which adjoin the passage openings in the direction of the interior space (6) and are intended for receiving the load to be treated.

2. Installation frame according to claim 1,
**characterised in that**
the front plate (4) insulates the interior space (6) in an airtight manner.

3. Installation frame according to claim 2,
**characterised in that**
the front plate (4) is of a sandwich construction with a plastics core (= insulating plate (22)) of thermally insulating material.

4. Installation frame according to claim 2,
**characterised by**
a double-walled front plate (4) having a thermally insulating vacuum between its walls (20, 21).

5. Installation frame according to any of claims 1 to 4,
**characterised in that**
the passage openings are each closed by an opening plate (5) corresponding in the internal structure thereof to the front plate (4).

6. Installation frame according to any of claims 1 to 5,
**characterised in that**
the shelves (7) are mounted on the front plate (4) so as to be longitudinally displaceable in the direction of the interior space (6).

7. Installation frame according to claim 6,
**characterised in that**
the opening plates (5) are fixed at the ends of the shelves (7) facing the front plate (4) in such a way that in each case a front plate (4) and a shelf (7) form a drawer with one another.

8. Installation frame according to any of claims 1 to 7,
**characterised in that**
the shelves (7) each consist of a holding frame and of one or more support plates (11) insertable into the holding frame.

9. Installation frame according to any of claims 1 to 8,
**characterised by**
two U-shaped support brackets (8) which are fixed with the end faces of their U-limbs (9) to the side of the front plate (4) facing the interior space (6) in such a way that the U-limb (9) facing the incubator base (10) of the incubator (1) carries runners on its outer side facing the incubator base (10).

10. Incubator (1) having an installation frame installed in its interior space (6) according to any of claims 1 to 9.

## Revendications

1. Cadre de montage à installer à l'intérieur (6) d'une étuve bactériologique (1), comprenant :
• un panneau frontal (4) qui garnit l'ouverture frontale de l'intérieur (6) ;
• au moins deux ouvertures de passage obturables vers l'intérieur (6) du panneau frontal (4) ; et
• des rayons (7) adjacents aux ouvertures de passage en direction de l'intérieur (6) pour la réception du matériau de charge à traiter.

2. Cadre de montage selon la revendication 1,
**caractérisé en ce que**
le panneau frontal (4) isole l'intérieur (6) de manière étanche à l'air.

3. Cadre de montage selon la revendication 2,
**caractérisé en ce que**
le panneau frontal (4) présente une structure sandwich avec un noyau en matière plastique (= plaque isolante [22]) en un matériau thermiquement isolant.

4. Cadre de montage selon la revendication 2,
**caractérisé par**
un panneau frontal (4) à double paroi avec un vide thermiquement isolant entre ses parois (20, 21).

5. Cadre de montage selon l'une des revendications 1 à 4,
**caractérisé en ce que**
les ouvertures de passage sont fermées chacune par une plaque à orifices (5) correspondant à la structure interne du panneau frontal (4).

6. Cadre de montage selon l'une des revendications 1 à 5,
**caractérisé en ce que**
les rayons (7) en direction de l'intérieur (6) sont disposés de manière déplaçable longitudinalement sur le panneau frontal (4).

7. Cadre de montage selon la revendication 6,
**caractérisé en ce que**
les plaques à orifices (5) sont fixées aux extrémités des rayons (7) tournées vers le panneau frontal (4) de telle sorte qu'un panneau frontal (4) et un rayon (7) forment ensemble un tiroir.

8. Cadre de montage selon l'une des revendications 1 à 7,
**caractérisé en ce que**
les rayons (7) comportent chacun un cadre de maintien et une ou plusieurs plaques de support (11) pouvant être encastrées dans le cadre de maintien.

9. Cadre de montage selon l'une des revendications 1 à 8,
**caractérisé par**
deux étriers de support (8) en forme de U fixés par les faces frontales de leurs branches en U (9) sur le côté du panneau frontal (4) tourné vers l'intérieur (6), tel que la branche en U (9) tournée vers le plancher de l'armoire (10) de l'étuve bactériologique (1) prend des patins de glissement sur son côté extérieur tourné vers le plancher de l'étuve bactériologique (10).

10. Étuve bactériologique (1) comportant un cadre de montage intégré dans son intérieur (6), selon l'une des revendications 1 à 9.
